# EUROPÄISCHE PATENTSCHRIFT

(11) **EP 3 311 744 B1**
(45) Veröffentlichungstag und Bekanntmachung des Hinweises auf die Patenterteilung: **26.02.2020**
(21) Anmeldenummer: 17180981.7
(22) Anmeldetag: 12.07.2017
(51) Int. Cl.: A61B 6/00

(54) **IN KOPF-FUSS-RICHTUNG ORTSAUFGELÖSTE WED-ERMITTLUNG**
SPATIALLY RESOLVED WED DETECTION IN HEAD-FOOT DIRECTION
DÉTERMINATION DE WED DÉCLENCHÉ PAR LOCATION EN DIRECTION TÊTE/PIED

(43) Veröffentlichungstag der Anmeldung: 25.04.2018
(73) Patentinhaber: Siemens Healthcare GmbH, 91052 Erlangen (DE)
(72) Erfinder: Dorn, Karlheinz, 90562 Kalchreuth (DE)

(56) Entgegenhaltungen:
- WO-A1-2017/103238
- US-A1- 2004 202 277
- US-A1- 2017 143 291
- Cynthia Mccollough ET AL: "Use of Water Equivalent Diameter for Calculating Patient Size and Size-Specific Dose Estimates (SSDE) in CT: The Report of AAPM Task Group 220", AAPM Rep. 2014, 30. September 2014 (2014-09-30), Seiten 6-23, XP055446368, Gefunden im Internet: URL:https://www.ncbi.nlm.nih.gov/pmc/artic les/PMC4991550/pdf/nihms-766038.pdf [gefunden am 2018-01-31]

## Beschreibung

Die vorliegende Erfindung geht aus von einem Auswertungsverfahren für eine Anzahl von mittels einer Röntgenanlage erfassten Röntgenbildern eines menschlichen Untersuchungsobjekts,
- wobei die Röntgenbilder jeweils in genau zwei Dimensionen ortsaufgelöst sind und die Arme des Untersuchungsobjekts enthalten,
- wobei sich, bezogen auf das Untersuchungsobjekt, jeweils eine Dimension in Kopf-Fuß-Richtung und eine weitere Dimension von links nach rechts erstreckt,
- wobei in den Röntgenbildern in Kopf-Fuß-Richtung äquidistanten Schritten jeweils die jeweilige Körperregion erkannt wird und hierauf aufbauend der auf Wasser bezogene äquivalente Durchmesser des Untersuchungsobjekts in einer zur Kopf-Fuß-Richtung orthogonalen Ebene ermittelt wird,
- wobei die für die jeweilige Position ermittelten äquivalenten Durchmesser unter Zuordnung zu den Röntgenbildern und der jeweiligen Position in Kopf-Fuß-Richtung abgespeichert werden.

Die vorliegende Erfindung geht weiterhin aus von einem Computerprogramm für eine Recheneinrichtung, wobei das Computerprogramm Maschinencode umfasst, der von der Recheneinrichtung abarbeitbar ist, wobei die Abarbeitung des Maschinencodes durch die Recheneinrichtung bewirkt, dass die Recheneinrichtung ein derartiges Auswertungsverfahren ausführt.

Die vorliegende Erfindung geht weiterhin aus von einer Recheneinrichtung, die mit einem derartigen Computerprogramm programmiert ist, so dass sie im Betrieb ein derartiges Auswertungsverfahren ausführt.

Die korrekte Ermittlung von im Rahmen von Röntgenuntersuchungen applizierten Dosen wird immer wichtiger. Im Stand der Technik wird für dreidimensionale Scans üblicherweise zunächst vorab ein zweidimensionales Röntgenbild (Topogramm) erfasst, wobei sich eine Dimension in Kopf-Fuß-Richtung des Untersuchungsobjekts erstreckt. In diesem Röntgenbild werden - automatisch oder spezifiziert durch einen Benutzer - bestimmte zweidimensionale Bereiche selektiert und für diese Bereiche wird einheitlich für den jeweiligen Bereich ein auf Wasser bezogener äquivalenter Durchmesser des Untersuchungsobjekts ermittelt. Anhand des zweidimensionalen Röntgenbildes erfolgt dann die Festlegung der Röntgendosis.

Die Vorgehensweise des Standes der Technik ist in verschiedener Hinsicht von Nachteil. So erfolgt zunächst eine Ermittlung der Durchmesser, die in Kopf-Fuß-Richtung nur sehr grob aufgelöst ist. Weiterhin ist eine Zuordnung zu bestimmten Tischpositionen, bei denen später Untersuchungen durchgeführt werden, nicht ohne weiteres erkennbar.

Ein Auswertungsverfahren der eingangs genannten Art ist beispielsweise aus der Veröffentlichung der American Association of Physicists in Medicine (2014): "Use of Water Equivalent Diameter for Calculating Patient Size and Size-Specific Dose Estimates (SSDE) in CT (Task Group 220)" bekannt.

Aus der US 2017/0 143 291 A1 ist ein Auswertungsverfahren für eine Anzahl von mittels einer Röntgenanlage erfassten Röntgenbildern eines menschlichen Untersuchungsobjekts bekannt,
- wobei die Röntgenbilder jeweils in genau zwei Dimensionen ortsaufgelöst sind und die Arme des Untersuchungsobjekts enthalten,
- wobei sich, bezogen auf das Untersuchungsobjekt, jeweils eine Dimension in Kopf-Fuß-Richtung und von links nach rechts erstreckt,
- wobei in den Röntgenbildern jeweils die jeweilige Körperregion erkannt wird und hierauf aufbauend der auf Wasser bezogene äquivalente Durchmesser des Untersuchungsobjekts in einer zur Kopf-Fuß-Richtung orthogonalen Ebene ermittelt wird.

Die aus dem Fachaufsatz bekannte Vorgehensweise arbeitet bereits recht gut. Sie ist jedoch noch verbesserungsfähig.

Die Aufgabe der vorliegenden Erfindung besteht darin, Möglichkeiten zu schaffen, mittels derer eine verbesserte Vorgehensweise zur Verfügung gestellt wird.

Die Aufgabe wird durch ein Auswertungsverfahren mit den Merkmalen des Anspruchs 1 gelöst. Vorteilhafte Ausgestaltungen des Auswertungsverfahrens sind Gegenstand der abhängigen Ansprüche 2 bis 9.

Erfindungsgemäß wird ein Auswertungsverfahren der eingangs genannten Art dadurch ausgestaltet, dass die Arme des Untersuchungsobjekts vor der Ermittlung der auf Wasser bezogenen äquivalenten Durchmesser aus dem Röntgenbild entfernt werden.

Der auf Wasser bezogene Durchmesser des Untersuchungsobjekts beschreibt die Gesamtschwächung von Röntgenstrahlung durch das Untersuchungsobjekt normiert auf die Schwächung von Wasser. Der auf Wasser bezogene Durchmesser des Untersuchungsobjekts kann insbesondere als der Durchmesser eines Wasserzylinders definiert sein, wobei der Wasserzylinder bei einer Computertomographiebildgebung die gleiche Gesamtschwächung der Röntgenintensität verursacht wie das Untersuchungsobjekt, wenn die Schnittebene der axialen Schnittbilder orthogonal zur Symmetrieachse des Wasserzylinders angeordnet ist. Der auf Wasser bezogene Durchmesser kann auch richtungsabhängig sein, insbesondere weil der Querschnitt des Untersuchungsobjekts im Regelfall nicht kreisförmig ist.

Zur Ermittlung des auf Wasser bezogenen Durchmessers werden vorzugsweise bei der jeweiligen Position in Kopf-Fuß-Richtung zunächst in der weiteren Dimension die Grenzen des Untersuchungsobjekts in mindestens einem der Röntgenbilder ermittelt. Sodann erfolgt zwischen den Grenzen eine Integration über die Hounsfield Units des jeweiligen Röntgenbildes. Der jeweilige auf Wasser bezogene äquivalente Durchmesser wird schließlich unter Verwendung des Ergebnisses der Integration ermittelt.

Manche Röntgenbilder sind zusätzlich zur Kopf-Fuß-Richtung in zwei weiteren Dimensionen ortsaufgelöst. In diesem Fall erfolgt im Stand der Technik die Ermittlung des auf Wasser bezogenen äquivalenten Durchmessers in einer einzelnen Schicht (slice) eines dreidimensionalen Scans. Die Schicht ist in diesem Fall orthogonal zur Kopf-Fuß-Richtung orientiert. Erfindungsgemäß ist das Röntgenbild bzw. mindestens eines der Röntgenbilder zusätzlich zur Kopf-Fuß-Richtung in nur einer einzigen weiteren Dimension ortsaufgelöst. Diese weitere Dimension erstreckt sich, bezogen auf das Untersuchungsobjekt, von links nach rechts (sogenanntes AP-Bild, wobei AP für "anterior posterior" steht). Im Falle einer Erstreckung, bezogen auf das Untersuchungsobjekt, von vorne nach hinten würde es sich um ein sogenanntes LAT-Bild handeln, wobei LAT für "lateral" steht). In beiden Fällen - also unabhängig davon, ob die einzige weitere Dimension sich von links nach rechts oder von vorne nach hinten erstreckt, - handelt es sich bei einem zweidimensionalen Röntgenbild um ein Topogramm.

In der Regel ist das Untersuchungsobjekt während der Erfassung der Röntgenbilder auf einem Patiententisch angeordnet. Zur verbesserten Ermittlung des auf Wasser bezogenen äquivalenten Durchmessers wird vorzugsweise der Patiententisch vor der Ermittlung der auf Wasser bezogenen äquivalenten Durchmesser aus mindestens einem der Röntgenbilder entfernt. Bei einem AP-Bild kann dies dadurch erfolgen, dass die Hounsfield Units des entsprechenden Röntgenbildes vor der Ermittlung des auf Wasser bezogenen äquivalenten Durchmessers um die prinzipiell bekannte Absorption des Patiententisches korrigiert werden.

Im Falle mindestens eines zweidimensionalen Röntgenbildes erfolgt oftmals weiterhin nach der Erfassung des zweidimensionalen Röntgenbildes bzw. der zweidimensionalen Röntgenbilder mittels der Röntgenanlage ein dreidimensionaler Scan des Untersuchungsobjekts. In diesem Fall können die anhand der zweidimensionalen Röntgenbilder ermittelten, auf Wasser bezogenen Durchmesser des Untersuchungsobjekts in Verbindung mit Einstellungen der Röntgenanlage bei der Durchführung des dreidimensionalen Scans zur Ermittlung eines auf die Größe des Untersuchungsobjekts bezogenen Dosiswertes (SSDE = size specific dose estimate) während des dreidimensionalen Scans verwendet werden. In diesem Fall kann weiterhin anhand der auf die Größe des Untersuchungsobjekts bezogenen Dosiswerte entschieden werden, ob an einen Benutzer der Röntgenanlage ein Alarm ausgegeben wird oder nicht. Dies vermeidet die oftmals überflüssigen Alarme, die im Stand der Technik auf Basis des sogenannten CTDIVOL ausgelöst werden.

Vorzugsweise wird bei der Ermittlung der auf Wasser bezogenen äquivalenten Durchmesser des Untersuchungsobjekts zusätzlich eine Position eines Patiententisches innerhalb einer orthogonal zur Kopf-Fuß-Richtung orientierten Ebene berücksichtigt, insbesondere eine Höhenlage des Patiententisches. Die hierfür erforderlichen Werte können alternativ von einem Benutzer vorgegeben werden oder automatisiert anhand von dem Röntgenbild zugeordneten Daten ermittelt werden.

Vorzugsweise werden im Rahmen des Auswertungsverfahrens nach dem Ermitteln die ermittelten, auf Wasser bezogenen äquivalenten Durchmesser des Untersuchungsobjekts durch Vergleich untereinander und/oder mit vorgegebenen oder anderweitig ermittelten Werten auf Plausibilität überprüft. Insbesondere können in Kopf-Fuß-Richtung aufeinanderfolgende ermittelte, auf Wasser bezogene äquivalente Durchmesser des Untersuchungsobjekts auf Sprünge untersucht werden und können weiterhin die ermittelten, auf Wasser bezogenen äquivalenten Durchmesser des Untersuchungsobjekts als solche oder in Verbindung mit der Position in Kopf-Fuß-Richtung anhand von vorbestimmten Werten auf Plausibilität geprüft werden.

Es ist möglich, die Auswertung anhand lokal gespeicherter Daten vorzunehmen. Vorzugsweise aber werden die Röntgenbilder aus einer Cloud abgerufen und/oder werden die auf Wasser bezogenen äquivalenten Durchmesser des Untersuchungsobjekts in der Cloud gespeichert.

Beim Cloud Computing stellt ein Anbieter Rechenleistung in Paketen zur Verfügung. Beispielsweise stehen einem Anbieter an einem bestimmten Ort auf der Erde insgesamt ca. 10.000 physikalische Rechner zur Verfügung. Jeder physikalische Rechner weist eine Anzahl von Rechnerkernen (CPUs) auf, beispielsweise 16 Rechnerkerne. Die physikalischen Rechner weisen weiterhin jeweils einen Arbeitsspeicher und einen Festplatten- oder vergleichbaren Speicher auf, beispielsweise 128 GB Arbeitsspeicher und 3 TB Festplattenspeicher. Die Rechnerkerne des jeweiligen Rechners teilen sich dynamisch den jeweiligen Arbeitsspeicher und den jeweiligen Festplattenspeicher. Die physikalischen Rechner können beispielsweise in 40-Fuß-Standardcontainern angeordnet sein. In mindestens einem weiteren derartigen Container ist ein zentraler Datenspeicher angeordnet, der beispielsweise mehrere 10 TB-Datenbank-Cluster umfasst, die jeweils aus einem System von mehreren Festplatten und spezialisierten Datenbank-Rechnern gebildet werden. Auch dieser Container ist an dem genannten Ort angeordnet. Die Container können permanent verschlossen bleiben. Sie benötigen lediglich Anschlüsse für die Energieversorgung, die Kühlung und die Kommunikation untereinander sowie mit dem Internet bzw. World Wide Web.

Der Anbieter bietet Gruppen von Rechnern an, die üblicherweise als virtuelle Maschinen bezeichnet werden. Von einem die virtuellen Maschinen verwaltenden Cloudrechner werden Anforderungen für virtuelle Maschinen entgegengenommen. Die Anforderungen werden von Rechnern von Rechnerverbunden gestellt und über das Internet an den Cloudrechner übermittelt. Der Cloudrechner reserviert daraufhin die jeweils angeforderte Menge an virtuellen Maschinen für die jeweilige Anforderung und übermittelt an den jeweils anfordernden Rechner die entsprechenden Zugriffsdaten, so dass vom jeweiligen Rechnerverbund aus auf die entsprechenden virtuellen Maschinen zugegriffen werden kann. Die Reservierung für die jeweilige Anforderung wird beibehalten, bis dem Cloudrechner vom anfordernden Rechner des Rechnerverbundes eine Freigabe übermittelt wird.

Das Auswertungsverfahren wird vorzugsweise vollautomatisch ausgeführt.

Die Aufgabe wird weiterhin durch ein Computerprogramm mit den Merkmalen des Anspruchs 10 gelöst. Erfindungsgemäß bewirkt die Abarbeitung des Maschinencodes durch die Recheneinrichtung, dass die Recheneinrichtung ein erfindungsgemäßes Auswertungsverfahren ausführt.

Die Aufgabe wird weiterhin durch eine Recheneinrichtung mit den Merkmalen des Anspruchs 11 gelöst. Erfindungsgemäß ist die Recheneinrichtung mit einem erfindungsgemäßen Computerprogramm programmiert, so dass sie im Betrieb ein erfindungsgemäßes Auswertungsverfahren ausführt.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: eine Röntgenanlage nebst zugehöriger Komponenten,
- FIG 2: ein AP-Bild,
- FIG 3: ein LAT-Bild,
- FIG 4: ein Schichtbild,
- FIG 5: ein Ablaufdiagramm,
- FIG 6: eine Linie eines Röntgenbildes,
- FIG 7: ein Ablaufdiagramm,
- FIG 8: ein Ablaufdiagramm und
- FIG 9: ein Ablaufdiagramm.

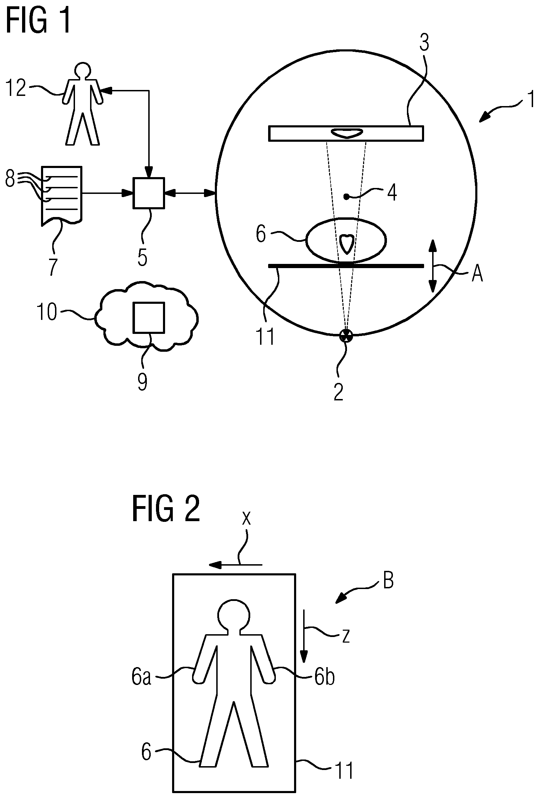

Gemäß FIG 1 weist eine Röntgenanlage 1 eine Röntgenquelle 2 und einen Röntgendetektor 3 auf. Die Röntgenquelle 2 und der Röntgendetektor 3 sind oftmals gemeinsam um eine Rotationsachse 4 rotierbar. Der Röntgendetektor 3 ist oftmals flächig ausgebildet, erfasst also ein zweidimensionales Bild. Mittels einer Recheneinrichtung 5 sollen mittels der Röntgenanlage 1 erfasste Röntgenbilder B eines menschlichen Untersuchungsobjekts 6 ausgewertet werden.

Die Recheneinrichtung 5 ist mit einem Computerprogramm 7 programmiert. Das Computerprogramm 7 umfasst Maschinencode 8, der von der Recheneinrichtung 5 abarbeitbar ist. Die Abarbeitung des Maschinencodes 8 durch die Recheneinrichtung 5 bewirkt, dass die Recheneinrichtung 5 ein nachstehend näher erläutertes Auswertungsverfahren ausführt.

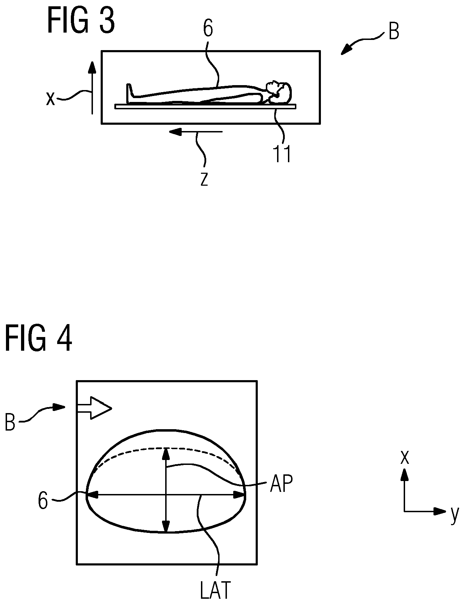

Die FIG 2 bis 4 zeigen verschiedene Röntgenbilder B. In allen Fällen ist das Röntgenbild B gemäß den FIG 2 bis 4 in mehreren Dimensionen ortsaufgelöst. Eine Dimension - nachfolgend als z-Richtung bezeichnet - erstreckt sich in Kopf-Fuß-Richtung des Untersuchungsobjekts 6. Wenn das Röntgenbild B zusätzlich zur z-Richtung in einer einzigen weiteren Dimension ortsaufgelöst ist, handelt es sich bei dem Röntgenbild B um ein Topogramm. In diesem Fall kann die weitere Richtung - nachfolgend x-Richtung bezeichnet - sich alternativ von links nach rechts oder von vorne nach hinten erstrecken. Derartige Röntgenbilder B sind Fachleuten allgemein bekannt und werden üblicherweise als AP-Bild bzw. als LAT-Bild bezeichnet. In beiden Fällen ist die weitere Dimension orthogonal zur z-Richtung. FIG 2 zeigt rein beispielhaft ein AP-Bild, FIG 3 ein LAT-Bild. Wenn das Röntgenbild B zusätzlich zur z-Richtung in zwei weiteren Dimensionen ortsaufgelöst ist, handelt es sich um ein Schichtbild eines dreidimensionalen Computertomogramms. Das Schichtbild als solches ist nur zweidimensional. Es ist jedoch dem größeren, dreidimensionalen Computertomogramm entnommen. FIG 4 zeigt rein beispielhaft ein derartiges Röntgenbild B. Die beiden weiteren Dimensionen sind in diesem Fall beide orthogonal zur z-Richtung und auch orthogonal zueinander. Sie sind in FIG 4 mit x und y bezeichnet. Die vorliegende Erfindung betrifft AP-Bilder.

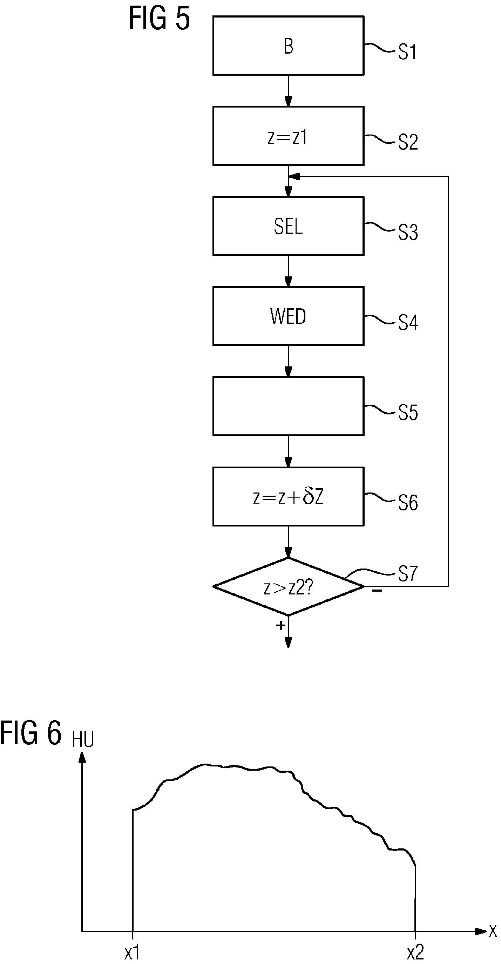

Zur Auswertung werden entsprechend der Darstellung in FIG 5 zunächst in einem Schritt S1 die Röntgenbilder B erfasst oder - falls die Röntgenbilder B bereits erfasst sind - die Röntgenbilder B aus einem Speicher 9 (siehe FIG 1) abgerufen. Der Speicher 9 ist vorzugsweise Bestandteil einer Cloud 10. In diesem Fall wird also das Röntgenbild B aus der Cloud 10 abgerufen.

In einem Schritt S2 setzt die Recheneinrichtung 5 die Position in z-Richtung auf einen Anfangswert z1. Der Anfangswert z1 ist vorzugsweise derart gewählt, dass er oberhalb des Kopfes des Untersuchungsobjekts 6 liegt.

In einem Schritt S3 selektiert die Recheneinrichtung 5 denjenigen Teil mindestens eines der Röntgenbilder B, der sich bei der entsprechenden Position in z-Richtung befindet. In einem Schritt S4 ermittelt die Recheneinrichtung 5 für die momentane Position in z-Richtung die relevante Körperregion des Untersuchungsobjekts 6, beispielsweise den Umriss des Kopfes, der Brust, des Bauchbereichs oder des Beckenbereichs. Hierauf aufbauend ermittelt die Recheneinrichtung 5 sodann für die relevante Körperregion des Untersuchungsobjekts 6 einen auf Wasser bezogenen äquivalenten Durchmesser WED des Untersuchungsobjekts 6. Die Ermittlung erfolgt in einer zur z-Richtung orthogonalen Ebene. Insbesondere kann die Recheneinrichtung 5 entsprechend der Darstellung in FIG 6 bei der jeweiligen Position in z-Richtung zunächst in der weiteren Dimension bzw. in den weiteren Dimensionen die Grenzen x1, x2 des Untersuchungsobjekts 6 in dem Röntgenbild B ermitteln und sodann zwischen den Grenzen x1, x2 eine Integration über die Hounsfield Units HU des Röntgenbildes B vornehmen. Das Ergebnis der Integration kann sodann ohne weiteres in den auf Wasser bezogenen äquivalenten Durchmesser WED des Untersuchungsobjekts 6 bei dieser Position in z-Richtung umgerechnet werden.

FIG 6 zeigt den Fall, dass das ausgewertete Röntgenbild B zweidimensional ist, sich also zusätzlich zur z-Richtung nur in einer einzigen weiteren Dimension erstreckt (in FIG 6 mit x bezeichnet). In diesem Fall sind die Grenzen x1, x2 des Untersuchungsobjekts 6 eine Funktion der Position in z-Richtung.

Die Recheneinrichtung 5 speichert den ermittelten, auf Wasser bezogenen äquivalenten Durchmesser WED in einem Schritt S5 unter Zuordnung zu den Röntgenbildern B und der jeweiligen Position in z-Richtung ab. Analog zum Abrufen der Röntgenbilder B im Schritt S1 ist auch hier eine lokale Speicherung möglich. Vorzugsweise erfolgt die Speicherung jedoch in der Cloud 10.

In einem Schritt S6 erhöht die Recheneinrichtung 5 die Position in z-Richtung um eine Schrittweite 5z. Die Schrittweite ist im Rahmen der Vorgehensweise von FIG 5 konstant. Sie liegt in der Regel im Bereich von 1 bis 2 mm, in manchen Fällen auch unter 1 mm. In einem Schritt S7 prüft die Recheneinrichtung 5, ob die Position in z-Richtung einen Endwert z2 erreicht hat bzw. überschritten hat. Der Endwert z2 ist in der Regel derart gewählt, dass er unterhalb der Füße des Untersuchungsobjekts 6 liegt.

Wenn der Endwert z2 noch nicht überschritten ist, geht die Recheneinrichtung 5 zum Schritt S3 zurück. Anderenfalls ist die Vorgehensweise von FIG 5 beendet.

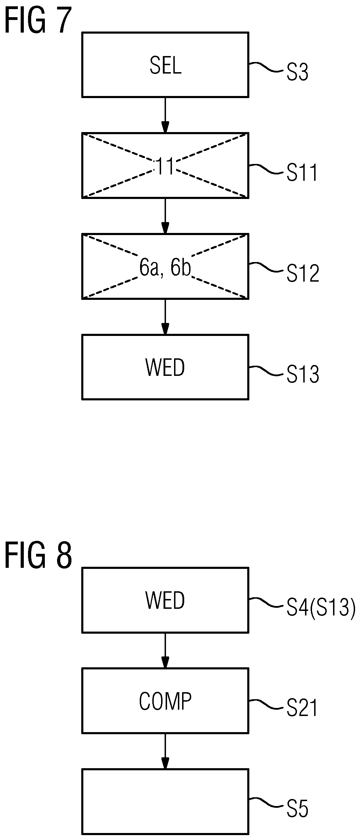

Wie aus den FIG 2 und 3 ersichtlich ist (und auch für FIG 4 gültig ist), ist das Untersuchungsobjekt 6 während der Erfassung der Röntgenbilder B auf einem Patiententisch 11 angeordnet. Vorzugsweise wird daher die Vorgehensweise von FIG 5 entsprechend der Darstellung in FIG 7 modifiziert.

Gemäß FIG 7 ist dem Schritt S4 ein Schritt S11 vorgeordnet. Im Schritt S11 wird der Patiententisch 11 aus dem ausgewerteten Röntgenbild B entfernt. Im Falle eines AP-Bildes (FIG 2) kann dies dadurch geschehen, dass die Pixelwerte der entsprechenden Linie des ausgewerteten Röntgenbildes B um die Abschwächung korrigiert werden, die durch den Patiententisch 11 hervorgerufen wird. Entsprechende Korrekturwerte können beispielsweise vorab ermittelt werden.

Bei einem AP-Bild (FIG 2) ist weiterhin entsprechend der Darstellung in FIG 7 ein Schritt S12 vorhanden, in dem auch die Arme 6a, 6b des Untersuchungsobjekts aus dem Röntgenbild B entfernt werden.

Im Rahmen der vorliegenden Erfindung ist der Schritt S12 stets vorhanden. Gegebenenfalls kann zusätzlich der Schritt S11 vorhanden sein.

Der Patiententisch 11 und hiermit verbunden auch das Untersuchungsobjekt 6 befinden sich orthogonal zur z-Richtung gesehen nicht stets bei derselben Position. Insbesondere ist es oftmals möglich, eine Höhenverstellung des Patiententischs 11 vorzunehmen, in FIG 1 durch einen Doppelpfeil A angedeutet. In manchen Fällen kann auch eine seitliche Verschiebung des Patiententischs 11 möglich sein. Die Höhenpositionierung des Patiententischs 11 und in geringerem Ausmaß auch die seitliche Positionierung des Patiententischs 11 haben Einfluss auf die Bestimmung des auf Wasser bezogenen äquivalenten Durchmessers WED. Alternativ oder zusätzlich zum Vorhandensein des Schrittes S11 ist es daher entsprechend der Darstellung in FIG 7 möglich, den Schritt S4 durch einen Schritt S13 zu ersetzen. Der Schritt S13 entspricht vom Ansatz her dem Schritt S4. Zusätzlich wird jedoch die Position des Patiententischs 11 innerhalb einer orthogonal zur z-Richtung orientierten Ebene berücksichtigt. Die entsprechenden Vorgaben können der Recheneinrichtung 5 nach Bedarf von einem Benutzer 12 (siehe FIG 1) vorgegeben werden oder Informationen sein, die den Röntgenbildern B zugeordnet sind und von der Recheneinrichtung 5 automatisch ausgewertet werden können.

Es ist möglich, die ermittelten, auf Wasser bezogenen äquivalenten Durchmesser WED so, wie sie ermittelt wurden, in dem Speicher 9 zu hinterlegen. Vorzugsweise ist jedoch dem Schritt S5 entsprechend der Darstellung in FIG 8 ein Schritt S21 vorgeordnet. Im Schritt S21 überprüft die Recheneinrichtung 5 die ermittelten, auf Wasser bezogenen äquivalenten Durchmesser WED des Untersuchungsobjekts 6 durch Vergleich untereinander und/oder mit vorgegebenen oder anderweitig ermittelten Werten auf Plausibilität.

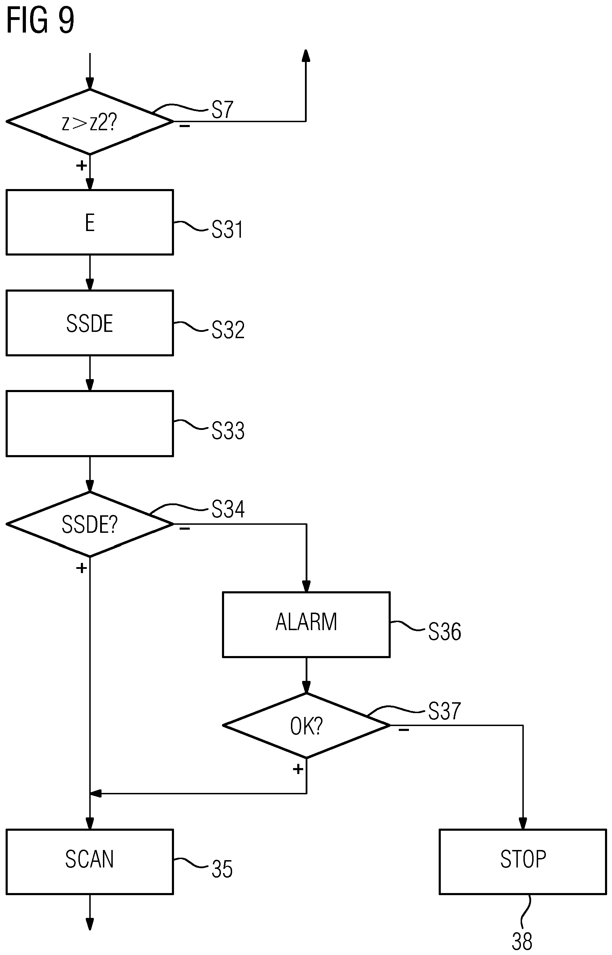

In vielen Fällen erfolgt nach der Erfassung mindestens eines zweidimensionalen Röntgenbildes B (also eines Topogramms) mittels der Röntgenanlage 1 ein dreidimensionaler Scan des Untersuchungsobjekts 6. Das Untersuchungsobjekt 6 verbleibt hierbei im Zeitraum zwischen dem Erfassen des mindestens einen Röntgenbildes B und dem Durchführen des dreidimensionalen Scans auf dem Patiententisch 11. In diesem Fall ist es möglich, die Vorgehensweise von FIG 5 entsprechend der Darstellung in FIG 9 zu erweitern und zu ergänzen.

Gemäß FIG 9 schließen sich an den Schritt S7 Schritte S31 bis S33 an. Im Schritt S31 werden der Recheneinrichtung 5 Einstellungen E der Röntgenanlage 1 bekannt, mit denen die Röntgenanlage 1 bei der Durchführung des dreidimensionalen Scans betrieben werden soll. Beispielsweise können die Einstellungen E der Recheneinrichtung 5 vom Benutzer 12 vorgegeben werden. Im Schritt S32 ermittelt die Recheneinrichtung 5 anhand der zuvor ermittelten, auf Wasser bezogenen Durchmesser WED des Untersuchungsobjekts 6 in Verbindung mit den Einstellungen E der Röntgenanlage einen Dosiswert SSDE. Der Dosiswert SSDE ist auf die Größe des Untersuchungsobjekts 6 bezogen. Er gibt an, in welchem Umfang das Untersuchungsobjekt 6 während des dreidimensionalen Scans mit Röntgenstrahlung belastet wird. Die Recheneinrichtung 5 gibt den ermittelten Dosiswert SSDE im Schritt S33 an den Benutzer 12 aus.

Zusätzlich können entsprechend der Darstellung in FIG 9 Schritte S34 bis S38 vorhanden sein. Im Schritt S34 prüft die Recheneinrichtung 5, ob an den Benutzer 12 ein Alarm ausgegeben werden soll. Die Prüfung des Schrittes S34 erfolgt durch Vergleich des ermittelten Dosiswertes SSDE mit einem vorbestimmten Grenzwert. Wenn kein Alarm ausgegeben werden soll, geht die Recheneinrichtung 5 zum Schritt S35 über. Im Schritt S35 wird der dreidimensionale Scan ausgeführt. Anderenfalls geht die Recheneinrichtung 5 zum Schritt S36 über. Im Schritt S36 wird der Alarm ausgegeben.

Im Schritt S37 prüft die Recheneinrichtung 5, ob ihr vom Benutzer 12 eine Quittierung vorgegeben wird. Wenn dies der Fall ist, geht die Recheneinrichtung 5 zum Schritt S35 über. Anderenfalls gibt die Recheneinrichtung 5 zum Schritt S38 über. Im Schritt S38 wird der dreidimensionale Scan vor seiner Ausführung abgebrochen.

Zusammengefasst betrifft die vorliegende Erfindung somit folgenden Sachverhalt:
Mittels einer Röntgenanlage 1 erfasste Röntgenbilder B eines menschlichen Untersuchungsobjekts 6 sind jeweils in mehreren Dimensionen ortsaufgelöst. Sie enthalten die Arme 6a, 6b des Untersuchungsobjekts 6. Jeweils eine Dimension erstreckt sich in Kopf-Fuß-Richtung des Untersuchungsobjekts 6, eine weitere Dimension erstreckt sich, bezogen auf das Untersuchungsobjekt 6, von links nach rechts. In den Röntgenbildern B wird in Kopf-Fuß-Richtung äquidistanten Schritten die jeweilige Körperregion erkannt und hierauf aufbauend jeweils der auf Wasser bezogene äquivalente Durchmesser WED des Untersuchungsobjekts 6 in einer zur Kopf-Fuß-Richtung orthogonalen Ebene ermittelt. Die für die jeweilige Position z ermittelten äquivalenten Durchmesser WED werden unter Zuordnung zu den Röntgenbildern B und der jeweiligen Position z in Kopf-Fuß-Richtung abgespeichert. Die Arme 6a, 6b des Untersuchungsobjekts 6 werden vor der Ermittlung der auf Wasser bezogenen äquivalenten Durchmesser WED aus diesem Röntgenbild B entfernt.

Die vorliegende Erfindung weist viele Vorteile auf. Insbesondere ist auf einfache und herstellerübergreifende Weise eine Bestimmung des auf Wasser bezogenen Durchmessers des Untersuchungsobjekts 6 möglich. Potentielle Fehlerquellen (Arme 6a, 6b, Patiententisch 11 usw.) können automatisch berücksichtigt werden. Eine Umrechnung in aussagekräftige SSDE-Werte und eine hierauf basierende Alarmprüfung sind möglich. Die Werte können insbesondere in der Cloud 10 hinterlegt werden und stehen dadurch langfristig und prinzipiell weltweit für statistische oder individuelle Auswertungen zur Verfügung. Der auf wasserbezogene Durchmesser WED des Untersuchungsobjekts 6 kann für jede Art von Röntgenbildern B (sowohl AP-Bilder als auch LAT-Bilder als auch Schichtbilder) ermittelt werden, und zwar in z-Richtung mit hoher Ortsauflösung.

Das erfindungsgemäße Auswertungsverfahren ist zu jedem Zeitpunkt ausführbar, sofern das zugrunde liegende Röntgenbild B bereits erfasst ist. Insbesondere kann die Auswertung bei einem Topogramm somit sowohl vor als auch nach der Durchführung eines nachfolgenden dreidimensionalen Scans erfolgen. Bei einem dreidimensionalen Scan kann die Auswertung selbstverständlich erst nach der Durchführung des Scans erfolgen. Die zugrundeliegenden Röntgenbilder können nach Bedarf lokal vorliegen, aus einem PACS abgerufen werden oder in der Cloud 10 gespeichert sein. Weiterhin können auch mehrere Röntgenbilder B gemeinsam ausgewertet werden. Beispielsweise können bei mindestens zwei zweidimensionalen Röntgenbildern B, von denen je mindestens eines ein AP-Bild und ein LAT-Bild ist, in z-Richtung gesehen in einem ersten Bereich eine Auswertung des AP-Bildes und in einem zweiten Bereich eine Auswertung des LAT-Bildes erfolgen. Der zweite Bereich kann hierbei insbesondere zum ersten Bereich komplementär sein. Auch andere kombinierte Auswertungen mehrerer Röntgenbilder B sind möglich.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Auswertungsverfahren für eine Anzahl von mittels einer Röntgenanlage (1) erfassten Röntgenbildern (B) eines menschlichen Untersuchungsobjekts (6),
- wobei die Röntgenbilder (B) jeweils in genau zwei Dimensionen ortsaufgelöst sind und die Arme (6a, 6b) des Untersuchungsobjekts (6) enthalten,
- wobei sich, bezogen auf das Untersuchungsobjekt (6), jeweils eine Dimension in Kopf-Fuß-Richtung und eine weitere Dimension von links nach rechts erstreckt,
- wobei in den Röntgenbildern (B) in Kopf-Fuß-Richtung äquidistanten Schritten jeweils die jeweilige Körperregion erkannt wird und hierauf aufbauend der auf Wasser bezogene äquivalente Durchmesser (WED) des Untersuchungsobjekts (6) in einer zur Kopf-Fuß-Richtung orthogonalen Ebene ermittelt wird,
- wobei die für die jeweilige Position (z) ermittelten äquivalenten Durchmesser (WED) unter Zuordnung zu den Röntgenbildern (B) und der jeweiligen Position (z) in Kopf-Fuß-Richtung abgespeichert werden,
**dadurch gekennzeichnet,**
**dass** die Arme (6a, 6b) des Untersuchungsobjekts (6) vor der Ermittlung der auf Wasser bezogenen äquivalenten Durchmesser (WED) aus dem Röntgenbild (B) entfernt werden.

2. Auswertungsverfahren nach Anspruch 1,
**dadurch gekennzeichnet,**
**dass** bei der jeweiligen Position (z) in Kopf-Fuß-Richtung zunächst in der weiteren Dimension bzw. in den weiteren Dimensionen die Grenzen (x1, x2, y1, y2) des Untersuchungsobjekts (6) in mindestens einem Röntgenbild (B) ermittelt werden, dass zwischen den Grenzen (x1, x2, y1, y2) eine Integration über die Hounsfield Units (HU) des jeweiligen Röntgenbildes (B) erfolgt und dass der jeweilige auf Wasser bezogene äquivalente Durchmesser (WED) unter Verwendung des Ergebnisses der Integration ermittelt wird.

3. Auswertungsverfahren nach Anspruch 1 oder 2,
**dadurch gekennzeichnet,**
**dass** das Untersuchungsobjekt (6) während der Erfassung der Röntgenbilder (B) auf einem Patiententisch (11) angeordnet ist und dass der Patiententisch (11) vor der Ermittlung der auf Wasser bezogenen äquivalenten Durchmesser (WED) aus mindestens einem der Röntgenbilder (B) entfernt wird.

4. Auswertungsverfahren nach Anspruch 1, 2 oder 3,
**dadurch gekennzeichnet,**
**dass** nach der Erfassung des zweidimensionalen Röntgenbildes (B) bzw. der zweidimensionalen Röntgenbilder (B) mittels der Röntgenanlage (1) ein dreidimensionaler Scan des Untersuchungsobjekts (6) erfolgt und dass die anhand der zweidimensionalen Röntgenbilder (B) ermittelten, auf Wasser bezogenen Durchmesser (WED) des Untersuchungsobjekts (6) in Verbindung mit Einstellungen (E) der Röntgenanlage (1) bei der Durchführung des dreidimensionalen Scans zur Ermittlung eines auf die Größe des Untersuchungsobjekts (6) bezogenen Dosiswertes (SSDE) während des dreidimensionalen Scans verwendet werden.

5. Auswertungsverfahren nach Anspruch 4,
**dadurch gekennzeichnet,**
**dass** anhand der auf die Größe des Untersuchungsobjekts (6) bezogenen Dosiswerte (SSDE) entschieden wird, ob an einen Benutzer (12) der Röntgenanlage (1) ein Alarm ausgegeben wird oder nicht.

6. Auswertungsverfahren nach einem der obigen Ansprüche,
**dadurch gekennzeichnet,**
**dass** bei der Ermittlung der auf Wasser bezogenen äquivalenten Durchmesser (WED) des Untersuchungsobjekts (6) eine Position eines Patiententisches (11) innerhalb einer orthogonal zur Kopf-Fuß-Richtung orientierten Ebene berücksichtigt wird.

7. Auswertungsverfahren nach einem der obigen Ansprüche,
**dadurch gekennzeichnet,**
**dass** die ermittelten, auf Wasser bezogenen äquivalenten Durchmesser (WED) des Untersuchungsobjekts (6) durch Vergleich untereinander und/oder mit vorgegebenen oder anderweitig ermittelten Werten auf Plausibilität überprüft werden.

8. Auswertungsverfahren nach einem der obigen Ansprüche,
**dadurch gekennzeichnet,**
**dass** die Röntgenbilder (B) aus einer Cloud (10) abgerufen werden und/oder dass die auf Wasser bezogenen äquivalenten Durchmesser (WED) des Untersuchungsobjekts (6) in der Cloud (10) gespeichert werden.

9. Auswertungsverfahren nach einem der obigen Ansprüche,
**dadurch gekennzeichnet,**
**dass** es vollautomatisch ausgeführt wird.

10. Computerprogramm für eine Recheneinrichtung (5), wobei das Computerprogramm Maschinencode (8) umfasst, der von der Recheneinrichtung (5) abarbeitbar ist, wobei die Abarbeitung des Maschinencodes (8) durch die Recheneinrichtung (5) bewirkt, dass die Recheneinrichtung (5) ein Auswertungsverfahren nach einem der obigen Ansprüche ausführt.

11. Recheneinrichtung, die mit einem Computerprogramm (7) nach Anspruch 10 programmiert ist, so dass sie im Betrieb ein Auswertungsverfahren nach einem der Ansprüche 1 bis 9 ausführt.

## Claims

1. Evaluation method for a number of X-ray images (B) of a human examination object (6) acquired by means of X-ray equipment (1),
- wherein the X-ray images (B) are each spatially resolved in exactly two dimensions and include the arms (6a, 6b) of the examination object (6),
- wherein, based on the examination object (6), in each case one dimension extends in the head-foot direction and a further dimension extends from left to right,
- wherein in each case the respective body region is detected in the X-ray images (B) in steps that are equidistant in the head-foot direction and, based on this, the water-equivalent diameter (WED) of the examination object (6) is determined in a plane orthogonal to the head-foot direction,
- wherein the water-equivalent diameters (WED) determined for the respective position (z) are stored with allocation to the X-ray images (B) and the respective position (z) in the head-foot direction,
**characterised in**
**that** the arms (6a, 6b) of the examination object (6) are removed from the X-ray image (B) before determination of the water-equivalent diameters (WED).

2. Evaluation method according to claim 1,
**characterised**
**in that** in the case of the respective position (z) in the head-foot direction, firstly the limits (x1, x2, y1, y2) of the examination object (6) are determined in at least one X-ray image (B) in the further dimension or in the further dimensions, in that integration over the Hounsfield Units (HU) of the respective X-ray image (B) takes place between the limits (x1, x2, y1, y2) and in that the respective water-equivalent diameter (WED) is determined using the result of integration.

3. Evaluation method according to claim 1 or 2,
**characterised**
**in that** during acquisition of the X-ray images (B), the examination object (6) is arranged on an examination table (11) and in that the examination table (11) is removed from at least one of the X-ray images (B) before determination of the water-equivalent diameters (WED).

4. Evaluation method according to claim 1, 2 or 3,
**characterised**
**in that** following acquisition of the two-dimensional X-ray image (B) or the two-dimensional X-ray images (B) by means of the X-ray equipment (1), a three-dimensional scan of the examination object (6) is carried out and in that the water-equivalent diameters (WED) of the examination object (6), determined using the two-dimensional X-ray images (B), are used during the three-dimensional scan in conjunction with settings (E) of the X-ray equipment (1) when performing the three-dimensional scan in order to determine a dose value (SSDE) based on the size of the examination object (6).

5. Evaluation method according to claim 4,
**characterised**
**in that** using the dose values (SSDE) based on the size of the examination object (6), a decision is made as to whether an alarm is output to a user (12) of the X-ray equipment (1) or not.

6. Evaluation method according to one of the above claims,
**characterised**
**in that** when determining the water-equivalent diameters (WED) of the examination object (6), a position of an examination table (11) within a plane oriented orthogonally to the head-foot direction is considered.

7. Evaluation method according to one of the above claims,
**characterised**
**in that** the determined, water-equivalent diameters (WED) of the examination object (6) are checked for plausibility by comparison with each other and/or with specified values or values determined in some other way.

8. Evaluation method according to one of the above claims,
**characterised**
**in that** the X-ray images (B) are retrieved from a cloud (10) and/or the water-equivalent diameters (WED) of the examination object (6) are stored in the cloud (10).

9. Evaluation method according to one of the above claims,
**characterised**
**in that** it is carried out completely automatically.

10. Computer program for an arithmetic device (5), wherein the computer program comprises machine code (8), which can be processed by the arithmetic device (5), wherein processing of the machine code (8) by the arithmetic device (5) causes the arithmetic device (5) to carry out an evaluation method according to one of the above claims.

11. Arithmetic device, which is programmed with a computer program (7) according to claim 10, so that during operation it carries out an evaluation method according to one of claims 1 to 9.

## Revendications

1. Procédé d'exploitation d'un nombre d'images (B) radiologiques détectées au moyen d'une installation (1) radiologique d'un objet (6) humain à examiner,
- dans lequel les images (B) radiologiques sont résolues spatialement chacune dans exactement deux dimensions et contiennent les bras (6a, 6b) de l'objet (6) à examiner,
- dans lequel, rapporté à l'objet (6) à examiner, une dimension s'étend dans la direction tête-pied et une autre dimension de gauche à droite,
- dans lequel, dans les images (B) radiologiques, on détecte, dans des pas équidistants dans la direction tête-pied, respectivement, les régions du corps et, en se fondant sur cela, on détermine le diamètre (WED) équivalent rapporté à l'eau de l'objet (6) à examiner dans un plan orthogonal à la direction tête-pied,
- dans lequel on mémorise les diamètres (WED) équivalents déterminés pour la position (z) respective, en association aux images (B) radiologiques et à la position (z) respective dans la direction tête-pied,
**caractérisé**
**en ce que** l'on élimine de l'image (B) radiologique les bras (6a, 6b) de l'objet (6) à examiner avant la détermination des diamètres (WED) équivalents rapportés à l'eau.

2. Procédé d'exploitation suivant la revendication 1, **caractérisé**
**en ce qu'**à la position (z) respective dans la direction tête-pieds on détermine d'abord, dans l'autre direction ou dans les autres directions, les limites (x1, x2, y1, y2) de l'objet (6) à examiner dans au moins une image (B) radiologique, **en ce que** l'on produit, entre les limites (x1, x2, y1, y2) une intégration sur les unités (HU) de Hounsfield de l'image (B) radiologique respective et **en ce que** l'on détermine le diamètre (WED) équivalent rapporté à l'eau respectif, en utilisant le résultat de l'intégration.

3. Procédé d'exploitation suivant la revendication 1 ou 2, **caractérisé**
**en ce que** l'objet (6) à examiner est disposé, pendant la détection des images (B) radiologiques, sur une couchette (11) de patient et **en ce que** l'on élimine d'au moins l'une des images (B) radiologiques la couchette (11) du patient, avant la détermination des diamètres (WED) équivalents rapportés à l'eau.

4. Procédé d'exploitation suivant la revendication 1, 2 ou 3, **caractérisé en ce qu'**après la détection de l'image (B) radiologique en deux dimensions ou des images (B) radiologiques en deux dimensions, on effectue, au moyen de l'installation (1) radiologique, un scan en trois dimensions de l'objet (6) à examiner et **en ce que** l'on utilise, pendant le scan en trois dimensions, pour la détermination d'une valeur (SSDE) de dose rapportée à la dimension de l'objet (6) à examiner, les diamètres (WED) rapportés à l'eau et déterminés à l'aide des images (B) radiologiques en deux dimensions de l'objet (6) à examiner, en liaison avec des réglages (E) de l'installation (1) radiologique lorsque l'on effectue le scan en trois dimensions.

5. Procédé d'exploitation suivant la revendication 4, **caractérisé en ce que**, à l'aide des valeurs (SSDE) de dose rapportées à la dimension de l'objet (6) à examiner, on décide si ou non on envoie une alerte à un utilisateur (12) de l'installation radiologique (1).

6. Procédé d'exploitation suivant l'une des revendications précédentes,
**caractérisé**
**en ce que**, lors de la détermination, des diamètres (WED) équivalents rapportés à l'eau de l'objet (6) à examiner, on prend en compte une position d'une couchette (11) de patient dans un plan orienté orthogonalement à la direction tête-pied.

7. Procédé d'exploitation suivant l'une des revendications précédentes,
**caractérisé**
**en ce que** l'on contrôle la vraisemblance de diamètres (WED) équivalents rapportés à l'eau de l'objet (6) à examiner, en les comparant entre eux et/ou à des valeurs données à l'avance ou déterminées d'une autre façon.

8. Procédé d'exploitation suivant l'une des revendications précédentes,
**caractérisé**
**en ce que** l'on extrait les images (B) radiologiques d'un nuage (10) et/ou **en ce que** l'on met en mémoire dans le nuage (10) les diamètres (WED) équivalents rapportés à l'eau de l'objet (6) à examiner.

9. Procédé d'exploitation suivant l'une des revendications précédentes,
**caractérisé**
**en ce que** on l'exécute d'une manière entièrement automatique.

10. Programme d'ordinateur pour un dispositif (5) de calcul, le programme d'ordinateur comprenant des codes (8) machine, qui peuvent être élaborés par le dispositif (5) de calcul, l'élaboration du code (8) machine, par le dispositif (5) de calcul, faisant que le dispositif (5) de calcul effectue un procédé d'exploitation suivant l'une des revendications précédentes.

11. Dispositif de calcul, qui est programmé par un programme (7) d'ordinateur suivant la revendication (10), de manière à effectuer en fonctionnement un procédé d'exploitation suivant l'une des revendications 1 à 9.
